# EUROPEAN PATENT APPLICATION

(11) **EP 3 753 507 A1**
(43) Date of publication of application: **23.12.2020**
(21) Application number: 17936937.6
(22) Date of filing: 29.12.2017
(51) Int. Cl.: A61B 17/34, A61B 8/00

(54) **SURGICAL NAVIGATION METHOD AND SYSTEM**

(71) Applicant: Weipeng (Suzhou) Medical Co., Ltd., Suzhou, Jiangsu 215123 (CN)
(72) Inventor: XIA, Yan, Suzhou, Jiangsu 215123 (CN); WANG, Pu, Suzhou, Jiangsu 215123 (CN); LAN, Lu, Suzhou, Jiangsu 215123 (CN)
(74) Representative: Manitz Finsterwald Patent- und Rechtsanwaltspartnerschaft mbB
(86) International application number: PCT/CN2017/120080
(87) International publication number: WO 2019/127449

(57) **Abstract**

A surgical navigation method and system (100). The method comprises: optically positioning an ultrasonic probe and puncture needle having been configured with an optical mark piece (S603); acquiring ultrasonic imaging data acquired in real time by the ultrasonic probe (S604); generating a position and trajectory of the puncture needle in an ultrasonic image according to optical positioning information and the ultrasonic imaging data (S605); and synchronously displaying the ultrasonic image, and the position and trajectory of the puncture needle in the ultrasonic image (S606). The system (100) comprises a positioning module (201), an ultrasonic imaging data acquisition module (202), a puncture needle trajectory generation module (203) and a display module (204). By means of the surgical navigation system (100), a puncture needle (101) and an ultrasonic probe (102) can be optically positioned, and a position and trajectory of the puncture needle (101) in an ultrasonic image (103) collected by the ultrasonic probe is displayed in real time; not only are the hazards of X-ray radiation eliminated for doctors and patients, but also the defects of fuzziness and non-intuitive guidance caused by traditional ultrasonic guidance are solved, so that the doctors can intuitively and efficiently learn the puncture direction in surgery.

## Description

### TECHNICAL FIELD

The present disclosure relates to the field of medical technology, and in particular, to a surgical navigation method and system.

### BACKGROUND

At present, minimally invasive surgery, such as percutaneous nephrolithotomy (PCNL), has become a popular treatment method due to its characteristics such as small trauma and rapid recovery. In minimally invasive surgery, its accurate and rapid surgical puncture paves the foundation for a successful surgery with reduced treatment time. In the course of minimally invasive surgery, the puncture guidance plays a leading role in achieving a good outcome. Currently, the most popular puncture guidance methods are mainly C-arm X-ray guidance, also known as fluoroscopy guidance, and ultrasound guidance. Yet the C-arm X-ray is radiative and induces radiation harms to the doctors and patients; and the ultrasound guidance has the problems of poor-resolution radiography and non-intuitive guidance of the puncture needle, which makes it difficult for doctors to handle the puncture direction and depth accurately.

### SUMMARY

It is necessary to provide a surgical navigation method and system to address the current problem of radiation risks of the puncture guiding method as well as the poor-resolution radiography and non-intuitive guidance of the puncture needle in the current minimally invasive surgery.

A surgical navigation method, which includes:
performing optical tracking on an ultrasound probe and a puncture needle configured with optical markers to acquire three-dimensional tracking information of the ultrasound probe and the puncture needle relative to a first coordinate system;
acquiring ultrasound imaging data collected in real time by the ultrasound probe, the ultrasound imaging data includes the ultrasound image relative to a second coordinate system;
according to the ultrasound image relative to the second coordinate system and the three-dimensional tracking information of the ultrasound probe and the puncture needle relative to the first coordinate system, generating a position and a trajectory of the puncture needle in the ultrasound image in the second coordinate system; and
synchronously displaying the ultrasound image and the position and the trajectory of the puncture needle in the ultrasound image in the second coordinate system.

In an embodiment, before performing optical tracking on the ultrasound probe and the puncture needle configured with the optical markers, the method further includes:
configuring the ultrasound probe and the puncture needle with the optical markers; and
acquiring three-dimensional calibration information of the ultrasound probe and the puncture needle configured with the optical markers; and
the performing optical tracking on the ultrasound probe and the puncture needle configured with the optical markers to acquire the three-dimensional tracking information of the ultrasound probe and the puncture needle relative to the first coordinate system includes:
according to the three-dimensional calibration information of the ultrasound probe and the puncture needle configured with the optical markers, performing optical tracking on the ultrasound probe and the puncture needle configured with the optical markers to acquire the three-dimensional tracking information of the ultrasound probe and the puncture needle relative to the first coordinate system.

In an embodiment, according to the ultrasound image relative to the second coordinate system and the three-dimensional tracking information of the ultrasound probe and the puncture needle relative to the first coordinate system, generating the position and the trajectory of the puncture needle in the ultrasound image in the second coordinate system includes:
according to the ultrasound image relative to the second coordinate system and the three-dimensional tracking information of the ultrasound probe and the puncture needle relative to the first coordinate system, transforming the coordinates of the puncture needle into the ultrasound image in the second coordinate system; and
generating the position and the trajectory of the puncture needle in the ultrasound image in the second coordinate system.

In an embodiment, the surgical navigation method further includes:
acquiring a mark of a target puncture area in the ultrasound image;
determining the shape of the target puncture area and a position of the target puncture area in the ultrasound image according to the mark; and
determining the distance between the puncture needle and the target puncture area in the ultrasound image according to the position of the target puncture area in the ultrasound image and the position and the trajectory of the puncture needle in the ultrasound image in the second coordinate system; and
the synchronously displaying the ultrasound image and the position and the trajectory of the puncture needle in the ultrasound image in the second coordinate system includes:
synchronously displaying the ultrasound image, the shape of the target puncture area, the position of the target puncture area in the ultrasound image, the distance between the puncture needle and the target puncture area, and the position and the trajectory of the puncture needle in the ultrasound image.

In an embodiment, the position of the puncture needle in the ultrasound image in the second coordinate system includes three-dimensional coordinates of the puncture needle in the ultrasound image in the second coordinate system, an offset distance of the puncture needle relative to the ultrasound image, and an intersection point between the extended trajectory of the puncture needle and an ultrasound imaging plane.

In an embodiment, the surgical navigation method further includes:
giving an alert if the offset distance of the puncture needle relative to the ultrasound image or the distance between the puncture needle and the target puncture area is smaller than a preset threshold.

A surgical navigation system, which includes:
a tracking module, configured to perform optical tracking on an ultrasound probe and a puncture needle configured with optical markers to acquire three-dimensional tracking information of the ultrasound probe and the puncture needle relative to the first coordinate system;
an ultrasound imaging data acquisition module, configured to acquire ultrasound imaging data collected in real time by the ultrasound probe, while the ultrasound imaging data includes an ultrasound image relative to the second coordinate system;
a puncture needle trajectory generating module, configured to generate, according to the ultrasound image relative to the second coordinate system and the three-dimensional tracking information of the ultrasound probe and the puncture needle relative to the first coordinate system, a position and a trajectory of the puncture needle in the ultrasound image in the second coordinate system; and
a display module, configured to synchronously display the ultrasound image and the position and the trajectory of the puncture needle in the ultrasound image in the second coordinate system.

In an embodiment, the surgical navigation system further includes:
an optical marker configuring module, configured to configure the ultrasound probe and the puncture needle with optical markers; and acquire three-dimensional calibration information of the ultrasound probe and the puncture needle configured with the optical markers.

In an embodiment, the surgical navigation system further includes:
a target puncture area determining module, configured to acquire a mark of a target puncture area in the ultrasound image;
the target puncture area determining module is further configured to determine a shape of the target puncture area and a position of the target puncture area in the ultrasound image according to the mark; and
the target puncture area determining module is further configured to determine a distance between the puncture needle and the target puncture area in the ultrasound image according to the position of the target puncture area in the ultrasound image and the position and the trajectory of the puncture needle in the ultrasound image in the second coordinate system.

In an embodiment, the position of the puncture needle in the ultrasound image in the second coordinate system includes an offset distance of the puncture needle relative to the ultrasound image and an intersection point between an extended trajectory of the puncture needle and an ultrasound imaging plane, and the surgical navigation system further includes:
an alert module, configured to give an alert when the offset distance of the puncture needle relative to the ultrasound image or the distance between the puncture needle and the target puncture area is smaller than a preset threshold.

### BRIEF DESCRIPTION OF THE DRAWINGS

FIG. 1 is a schematic view of an application of a surgical navigation system in an embodiment.
FIG. 2 is a schematic view of a structure of a surgical navigation system in an embodiment.
FIG. 3 is a schematic view of a structure of a surgical navigation system in an embodiment.
FIG. 4 is a schematic view of a structure of a surgical navigation system in an embodiment.
FIG. 5 is a schematic view of a structure of a surgical navigation system in an embodiment.
FIG. 6 is a flowchart of a surgical navigation method in an embodiment.
FIG. 7 is a schematic view of a coordinate system of a surgical navigation method with a tracking module as a reference in an embodiment.
FIG. 8 is a schematic view of a coordinate system of a surgical navigation method with an ultrasound imaging plane as a reference in an embodiment.
FIG. 9 is a schematic diagram of navigation display of a surgical navigation method in an embodiment.

### DETAILED DESCRIPTION

To clearly represent the objectives, technical solutions and advantages of the embodiments of the present disclosure, the following clearly and completely describes the technical solutions in the embodiments of the present disclosure with reference to the accompanying drawings in the embodiments of the present disclosure. Apparently, the described embodiments are part rather than all of the embodiments of the present disclosure.

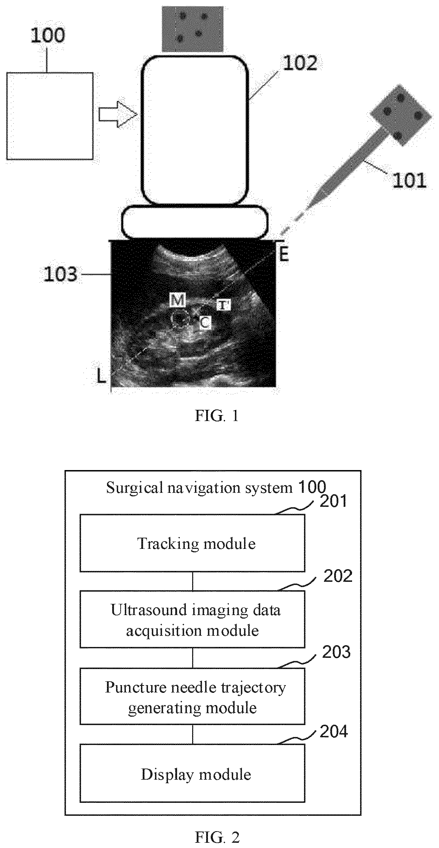

An embodiment of the present disclosure provides a surgical navigation system. In a specific application scenario, as shown in FIG. 1, for example, in a minimally invasive surgery, by means of the surgical navigation system 100, the puncture needle 101 and the ultrasound probe 102 can be optically positioned, and the position (as shown by *T'* and cross point C in FIG. 1, where *T'* is a projection of a tip of the puncture needle on the ultrasound imaging plane, the cross point C is the intersection point between the extended trajectory of the puncture needle and the ultrasound imaging plane) and the trajectory (the dashed line segment *EL* in FIG. 1, that is, a projection of the puncture needle on the ultrasound imaging plane and its extension line segment) of the puncture needle 101 and the target puncture area (the area M in FIG. 1 surrounded by a curve) in the ultrasound image 103 collected by the ultrasound probe 102 can be displayed in real time, which not only eliminates the X-ray radiation hazard to the doctor and patient, but also resolves the disadvantages of poor resolution radiography and non-intuitive guidance in the conventional ultrasound guided radiography, so that the doctor can handle the puncture direction of the surgery intuitively and efficiently.

In an embodiment, as shown in FIG. 2, the surgical navigation system 100 may include a tracking module 201, an ultrasound imaging data acquisition module 202, a puncture needle trajectory generating module 203, and a display module 204.

The tracking module 201 is configured to perform optical tracking on an ultrasound probe and a puncture needle configured with optical markers to acquire three-dimensional tracking information of the ultrasound probe and the puncture needle relative to a first coordinate system.

In this embodiment, the optical marker may be an active light-emitting or optical reflective device. The first coordinate system is a coordinate system with the tracking module 201 as reference. The present embodiment acquires three-dimensional tracking information of the ultrasound probe and the puncture needle in the first coordinate system by configuring a device required for surgery, such as the ultrasound probe and the puncture needle, with optical markers, so as to optically position the ultrasound probe and the puncture needle configured with the optical markers by the tracking module 201. The three-dimensional tracking information includes three-dimensional coordinates and directions of the ultrasound probe and the puncture needle in the first coordinate system, and its tracking error can be smaller than 2 mm.

The ultrasound imaging data acquisition module 202 is configured to acquire ultrasound imaging data collected in real time by the ultrasound probe. The ultrasound imaging data includes the ultrasound image relative to the second coordinate system. In this embodiment, the second coordinate system is a coordinate system that uses the ultrasound imaging plane as a reference. In this embodiment, the ultrasound imaging data acquisition module 202 may, in real time, read the ultrasound imaging data collected by the ultrasound probe by using a data interface of the ultrasonic machine itself, an on-board interface of the ultrasonic machine, or a medical information network server interface. The ultrasound imaging data includes but is not limited to ultrasound images, timestamp data, working status data, etc.

The puncture needle trajectory generating module 203 is configured to generate position and trajectory of the puncture needle in the ultrasound image in the second coordinate system according to the ultrasound image relative to the second coordinate system and the three-dimensional tracking information of the ultrasound probe and the puncture needle relative to the first coordinate system.

In this embodiment, according to the three-dimensional coordinates and directions of the ultrasound probe and the puncture needle in the first coordinate system and the ultrasound image relative to the second coordinate system collected in real time by the ultrasound probe, the three-dimensional coordinate and direction relative to the first coordinate system of the puncture needle are transformed into the ultrasound image in the second coordinate system, thereby generating the position and the trajectory of the puncture needle in the ultrasound image in the second coordinate system.

The display module 204 is configured to synchronously display the ultrasound image and the position and the trajectory of the puncture needle in the ultrasound image in the second coordinate system.

The position of the puncture needle in the ultrasound image in the second coordinate system includes quantitative information such as the offset angle and offset distance between the puncture needle and the ultrasound image. The trajectory of the puncture needle in the ultrasound image in the second coordinate system includes the projection and the extended trajectory of the puncture needle in the ultrasound image. Such that doctors can intuitively handle the direction of puncture via the contents displayed in the display module 204, thereby improving the accuracy and efficiency of puncturing.

In a specific embodiment, the display module 204 includes but is not limited to several display screens, where the display screen may be a normal display screen or a touchable display screen.

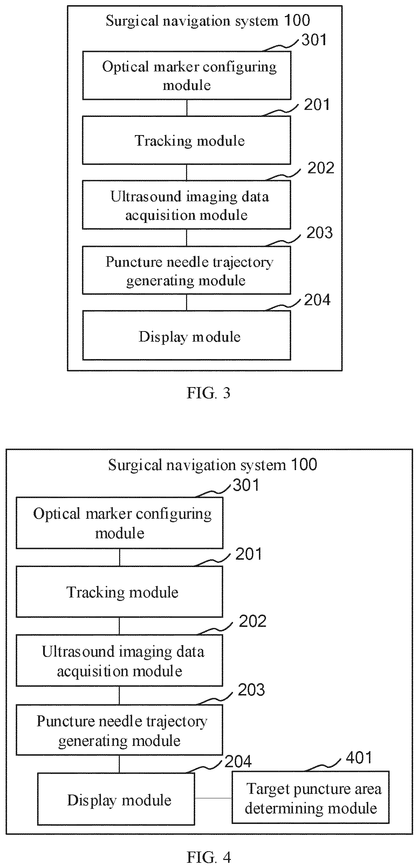

In an embodiment, as shown in FIG. 3, in the surgery process, in order to enable the tracking module 201 to accurately track the ultrasound probe and the puncture needle, the surgical navigation system 100 in this embodiment may therefore further include an optical marker configuring module 301 configured to configure the ultrasound probe and the puncture needle with optical markers, which may be specifically configured at tails of the ultrasound probe and the puncture needle, so that the tracking module 201 can optically identify, track, and track the ultrasound probe and the puncture needle configured with the optical markers. In this embodiment, the optical marker may be composed of three or more active light-emitting or reflective optical marking parts. For example, it may be an active light-emitting LED or a reflective ball (whose illumination light source may be provided by the tracking module 201). The active light-emitting LED or light source and reflection band are preferably infrared bands.

In this embodiment, the optical marker configuring module 301 may further acquire three-dimensional calibration information of the ultrasound probe and the puncture needle configured with optical markers. The three-dimensional calibration information refers to the three-dimensional shape of the rigid body formed after the optical markers are fixed to the ultrasound probe or puncture needle and the positional relationship between the components, which can be specifically determined by the computer-aided design (CAD) file. Specifically, the three-dimensional calibration information may include but is not limited to the starting position of imaging, imaging depth, imaging resolution, and imaging size of the ultrasound probe, the three-dimensional shapes of the ultrasound probe and the puncture needle itself, the positional relationship among the components, and so on.

In an embodiment, as shown in FIG. 4, the surgical navigation system 100 may further include a target puncture area determining module 401 configured to acquire a marker of the target puncture area in the ultrasound image, the target puncture area determining module 401 may further determine the shape of the target puncture area and the position of the target puncture area in the ultrasound image according to the mark, and determine the distance between the puncture needle and the target puncture area in the ultrasound image according to the position of the target puncture area in the ultrasound image and the position and the trajectory of the puncture needle in the second coordinate system.

In this embodiment, the doctor may mark the target puncture area in the ultrasound image according to the displayed ultrasound image. Specifically, the doctor may mark the position of the puncture target in the ultrasound image by using a mouse or touching gesture on a touchscreen. Therefore, the surgical navigation system 100 can further acquire the mark of the target puncture area by the doctor through the target puncture area determining module 401, so that the shape of the target puncture area and the position of the target puncture area in the ultrasound image can be determined according to the shape and the region of the mark, and then the distance between the puncture needle and the target puncture area in the ultrasound image can be determined according to the position of the target puncture area in the ultrasound image and the position and the trajectory of the puncture needle in the ultrasound image in the second coordinate system, and the distance and the corresponding trajectory can be displayed.

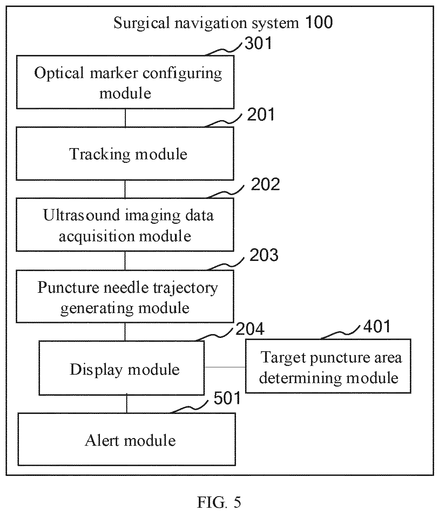

In an embodiment, the position of the puncture needle in the ultrasound image in the second coordinate system includes quantitative information such as offset distance, offset angle, etc., of the puncture needle relative to the ultrasound image. Thus, in this embodiment, as shown in FIG. 5, the surgical navigation system 100 may further include an alert module 501 configured to give an alert when the offset distance of the puncture needle relative to the ultrasound image or the distance between the puncture needle and the target puncture area is smaller than a preset threshold. Specifically, the way of alerting can be sound alert, light alert and so on, so that doctors can handle the direction and depth of puncture more intuitively, which further improves the accuracy and efficiency of puncturing.

The surgical navigation system provided in this embodiment does not require a separate ultrasonic device, can be adapted to work cooperatively with the existing ultrasonic machine and ultrasound probe, and does not change the handheld manner of the ultrasound probe. Therefore, the surgical navigation system will greatly improve the accuracy and efficiency of the surgical puncture at a low cost.

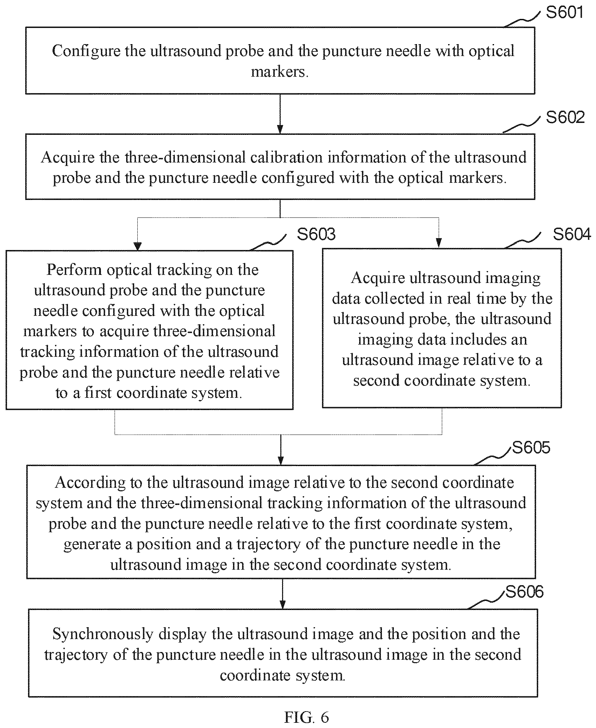

An embodiment of the present disclosure provides a surgical navigation method. The described surgical navigation system may be the executing body of the surgical navigation method. As shown in FIG. 6, the method includes the following steps:

Step S601: configure the ultrasound probe and the puncture needle with optical markers.

In this embodiment, the optical marker may be composed of three or more active light-emitting or reflective optical marking parts. Optical tracking of the ultrasound probe and the puncture needle in the subsequent surgery is facilitated by the configuration of optical markers on devices needs to be used such as the ultrasound probe and the puncture needle prior to surgery.

Step S602: acquire the three-dimensional calibration information of the ultrasound probe and the puncture needle configured with the optical markers.

The three-dimensional calibration information refers to the three-dimensional shape of the rigid body formed after the optical markers are fixed to the ultrasound probe or the puncture needle and the positional relationship between the components, which can be specifically determined by CAD design. Specifically, the three-dimensional calibration information may include but is not limited to the starting position of imaging, imaging depth, imaging resolution, and imaging size of the ultrasound probe, the three-dimensional shapes of the ultrasound probe and the puncture needle, the positional relationship among the components, and so on.

Step S603: perform optical tracking on the ultrasound probe and the puncture needle configured with the optical markers to acquire three-dimensional tracking information of the ultrasound probe and the puncture needle relative to a first coordinate system.

In this embodiment, the three-dimensional tracking information of the ultrasound probe and the puncture needle in the first coordinate system is acquired by optically tracking the ultrasound probe and the puncture needle configured with the optical markers. The three-dimensional tracking information includes the three-dimensional coordinates and directions of the ultrasound probe and the puncture needle in the first coordinate system. The first coordinate system is a coordinate system with the tracking module 201 as reference.

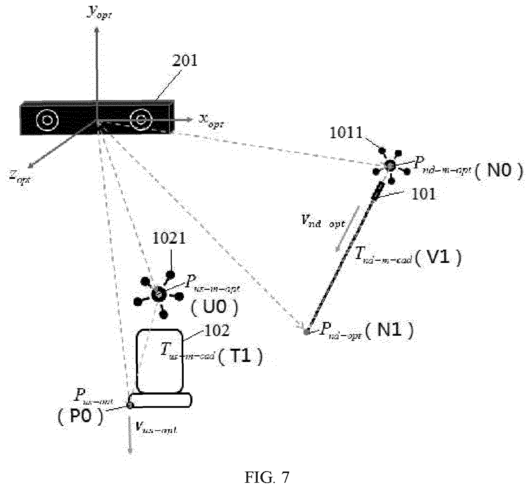

In a specific embodiment, as shown in FIG. 7, assuming that the coordinate axis of the tracking module 201 is *xₒₚₜ,yₒₚₜ,zₒₚₜ,* the three-dimensional tracking information of the ultrasound probe and the puncture needle in the coordinate system includes:

*Pᵤₛ₋ₘ₋ₒₚₜ*, which is, in a reference system of the tracking module 201, the three-dimensional coordinates of a geometric center of an optical tracking mark 1021 mounted on the ultrasound probe 102, as shown the central point U0 of the optical tracking mark 1021 in FIG. 7;

*T_{us-m-cad}*, which is, in a three-dimensional CAD design, the relative position relationship between the origin of the imaging plane of the ultrasound probe 102 and the geometric center of the optical tracking mark 1021 mounted on the ultrasound probe 102, as shown the T1 dashed line segment (i.e., U0-P0) in FIG. 7;

*Pᵤₛ₋ₒₚₜ*, which is, in the reference system of the tracking module 201, three-dimensional coordinates of the origin of the imaging plane of the ultrasound probe 102, which may be understood as *Pᵤₛ₋ₒₚₜ* = *Pᵤₛ₋ₘ₋ₒₚₜ* + *R_{cad-opt}* ∗ *T_{us-m-cad}* (the *R_{cad-opt}* is a coordinate conversion relationship between the reference system of the three-dimensional CAD design and the reference system of the tracking module 201), as shown the point P0 in FIG. 7;

*Vᵤₛ₋ₒₚₜ*, which is, in the reference system of the tracking module 201, the direction of the ultrasound probe 102 (as indicated by the arrow);

*P_{nd-m-opt}*, which is, in the reference system of the tracking module 201, the three-dimensional coordinates of the optical tracking mark 1011 mounted on the puncture needle 101, as shown the central point NO of the optical tracking mark 1011 in FIG. 7;

*T_{nd-m-cad}*, which is, in the three-dimensional CAD design, the relative positional relationship between the tip of the puncture needle 101 and the geometric center of the optical tracking mark 1011 mounted on the puncture needle 101, as shown the line segment V1 (i.e., N0-N1) in FIG. 7;

*P_{nd-opt}*, which is, in the reference system of the tracking module 201, the three-dimensional coordinates of the tip of the puncture needle 101, which may be understood as *P_{nd-opt}* = *P_{nd-m-opt}* + *R_{cad-opt}* ∗ *T_{nd-m-cad}* (*R_{cad-opt}* is the coordinate conversion relationship between the reference system of three-dimensional CAD design and the reference system of the tracking module 201), as shown the point N1 in FIG. 7; and

*V_{nd-opt}*, which is, in the reference system of the tracking module 201, the direction of the puncture needle 101 (as indicated by the arrow).

Step S604: acquire ultrasound imaging data collected in real time by the ultrasound probe, the ultrasound imaging data includes an ultrasound image relative to a second coordinate system.

Ultrasound imaging data includes but is not limited to ultrasound images, timestamp data, working status data, etc. The second coordinate system is a coordinate system with ultrasound imaging plane as reference.

Step S605: according to the ultrasound image relative to the second coordinate system and the three-dimensional tracking information of the ultrasound probe and the puncture needle relative to the first coordinate system, generate a position and a trajectory of the puncture needle in the ultrasound image in the second coordinate system.

Specifically, according to the ultrasound image relative to the second coordinate system and the three-dimensional tracking information of the ultrasound probe and the puncture needle relative to the first coordinate system, the coordinates of the puncture needle are transformed into the ultrasound image relative to the second coordinate system, so that the position and the trajectory of the puncture needle in the ultrasound image relative to the second coordinate system are generated.

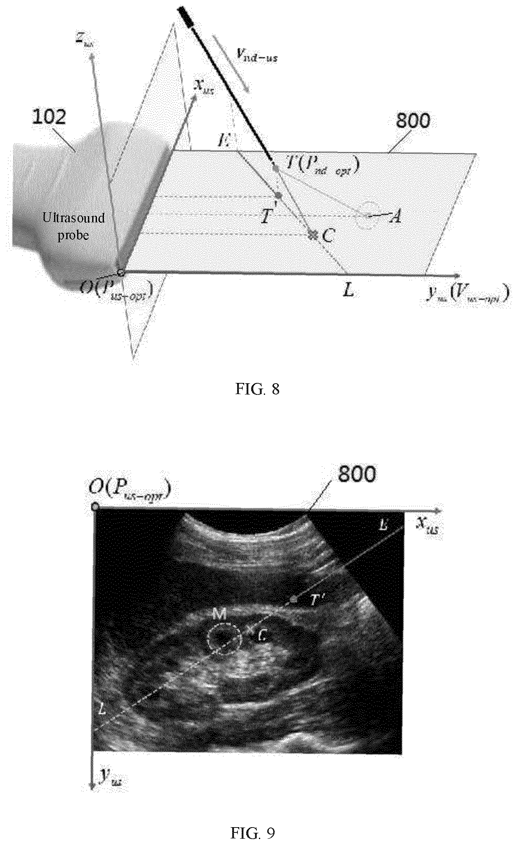

In an embodiment, as shown in FIG. 8, it is assumed that the ultrasound imaging plane 800 is used as a reference system, and its corresponding coordinate axis is *xᵤₛ*, *yᵤₛ, zᵤₛ,* where *yᵤₛ* is the *Vᵤₛ₋ₒₚₜ* in FIG. 7 (i.e., the direction of the ultrasound probe 102);

*0* is the starting point of a reference system of ultrasound imaging plane, that is, the point P0 of *Pᵤₛ₋ₒₚₜ* in FIG. 7 (the three-dimensional coordinates of the image collecting starting point of the ultrasound probe 102), also, the size of the ultrasound imaging plane 800 may be determined by the imaging depth, imaging resolution, and imaging size of the ultrasound probe 102 in the three-dimensional calibration information.

*V_{nd-us}* is, in the reference system of ultrasound imaging plane, the direction of the puncture needle 101, which can be understood as *V_{nd-us}* = *V_{nd-opt}* - *Vᵤₛ₋ₒₚₜ*.

*T* is, in the reference system of the ultrasound imaging plane, the three-dimensional coordinates of the tip of the puncture needle, that is, the point *P_{nd-opt}* in FIG. 7, which may be understood as *T* = *Pₙₐ₋ₒₚₜ* - *Pᵤₛ₋ₒₚₜ*.

*T'* is the projection of the tip *T* of the puncture needle on the ultrasound imaging plane.

*TT'* is, in the reference system of the ultrasound imaging plane, the positional relationship between the tip *T* of the puncture needle and the projection *T'* of the tip *T,* its length is the offset distance of the puncture needle relative to the ultrasound imaging plane.

*ET'* is the projection of the puncture needle in the ultrasound imaging plane.

*E* is the intersection point between the projection of the puncture needle in the ultrasound imaging plane and the edge of the ultrasound imaging plane, which can be understood as an accessing point of the projection *ET'* of the puncture needle accessing the ultrasound imaging plane.

*T'L* is the extended trajectory of the puncture needle projection *ET'.*

*L* is the intersection point between the projection extension line *T'L* of the puncture needle and the edge of the ultrasound imaging plane, which can be understood as the departing point of the projection of the puncture needle and the extension line segment *EL* of the projection departing from the ultrasound imaging plane.

*EL* is the projection of the puncture needle in the ultrasound image and the extension line of the projection, which is the trajectory of the puncture needle in the ultrasound image.

*C* is the intersection point between the extended trajectory of the puncture needle and the ultrasound imaging plane, located on the extension line *T'L* of the projection of the puncture needle, as shown in the gray cross in FIG. 8.

*TCT'* is, in the reference system of the ultrasound imaging plane, the angle between the direction of the puncture needle and the ultrasound imaging plane, whose angle value is the offset angle of the puncture needle relative to the ultrasound imaging plane.

*A* is the geometric central point of the puncture target area (circle area in the figure) on the ultrasound imaging plane, whose coordinates on the ultrasound imaging plane are determined by image recognition (the initial position is manually marked, and then the position is tracked by image recognition);

*AT* is, in the reference system of the ultrasound imaging plane, the positional relationship between the geometric center *A* of the puncture target area and the tip *T* of the puncture needle, whose length is the distance between the tip of the puncture needle and the puncture target area.

Step S606: synchronously display the ultrasound image and the position and the trajectory of the puncture needle in the ultrasound image in the second coordinate system.

The present disclosure will be further illustrated through the specific embodiment as follows. Generally, before performing the surgical puncture navigation, an optical tracking mark 1021 is mounted on the tail of the medical ultrasound probe 102 and an optical tracking mark 1011 is mounted on the tail of the medical puncture needle 101 by an optical marker configuring module, and then three-dimensional calibration information of the ultrasound probe and the puncture needle and their optical tracking marks is collected, calculated, and stored, where the three-dimensional calibration information refers to coordinate information of the ultrasound probe and the puncture needle and their optical tracking marks in the CAD design.

In this embodiment, the optical marker configuring module transmits the three-dimensional calibration information of the ultrasound probe and the puncture needle and their optical tracking marks to the tracking module, and performs surgical puncture navigation after completing the calibration. Specifically, optical tracking may be performed by a tracking module, that is, by collecting optical signals of the optical tracking marks 1011 and 1021, three-dimensional tracking information of the puncture needle 101 and the ultrasound probe 102 is parsed according to the collected optical signals, where the three-dimensional tracking information refers to coordinate information of the puncture needle 101 and the ultrasound probe 102 in a coordinate system with the tracking module as reference system.

At the same time, the ultrasound imaging data collected in real time by the ultrasound probe is acquired by the ultrasound imaging data acquisition module, the ultrasound imaging data may specifically include ultrasound image, time stamp data, and working status data, etc. Then, according to the three-dimensional tracking information of the puncture needle 101 and the ultrasound probe 102 and the ultrasound image, the puncture needle trajectory generating module transforms the coordinates of the puncture needle into the ultrasound image in the second coordinate system, where the second coordinate system is a coordinate system with the ultrasound image as reference. The position and the trajectory of the puncture needle in the ultrasound image are therefore calculated and displayed by the display module. The position and the trajectory of the puncture needle in the ultrasound image include quantitative information such as the offset angle and offset distance of the puncture needle relative to the ultrasound image.

In this embodiment, the target puncture area may also be marked in the displayed ultrasound image, so that the mark of the area is acquired by the target puncture area determining module, as the circular area M shown in FIG. 9, where the shape of the area may be set differently according to actual needs. Thus, the position of the target puncture area in the ultrasound image 800 can be determined according to the mark, and the distance between the puncture needle and the target puncture area in the ultrasound image can be determined by combining the position and the trajectory of the puncture needle in the ultrasound image in the second coordinate system. When the distance between the puncture needle and the target puncture area is smaller than a preset threshold, an alert can be further given by an alert module. In this embodiment, when the offset distance of the puncture needle relative to the ultrasound image is smaller than a preset threshold, an alert may also be given by the alert module. Thereby clearly indicating the direction and depth of the puncture and the position of the target puncture area, which is beneficial to the accurate and rapid completion of the puncture.

As shown in FIG. 9, the line segment ET' and the line segment T'L form the trajectory of the puncture needle in the ultrasound imaging plane, the cross C is the intersection point between the direction of the puncture needle and the ultrasound imaging plane, the curve M is the outline of the target puncture area, and the mass shadow in the outline is the puncture target. The currently displayed picture shows that the trajectory of the puncture needle in the ultrasound imaging plane (i.e., line segment EL) passing through the target puncture area (i.e., curve M), but the predicted position (i.e., the cross C) of the ultrasound imaging plane the puncture needle is about to pass through is outside the target area. Therefore, by adjusting the puncture needle, the cross C can be kept in the target area (i.e., M), and the puncture needle is pushed until the cross C overlaps with the tip (i.e., T') of the puncture needle, then the navigation can be completed and the puncture is successfully achieved.

In the foregoing embodiment, in order to enhance the display effect and make the display result more intuitive, the ultrasound image displayed in the display module 204 and the position and the trajectory of the puncture needle in the ultrasound image may be displayed in color. Specifically, the trajectory of the puncture needle and the extension line of the trajectory in the ultrasound image may be distinguished by line segments with different shapes, thicknesses, or colors, respectively, so that the doctor can view the surgical navigation screen more efficiently.

The technical features of the foregoing embodiments may be combined arbitrarily. For brevity of description, not all possible combinations of the technical features of the foregoing embodiments are described. However, to the extent the combinations of these technical features are not contradictory, they should be considered as within the scope of the specification.

The foregoing embodiments express only a few embodiments of the present disclosure, and the description thereof is more specific and detailed, but may not thus be construed as limiting the scope of the present disclosure. It should be noted that a person with ordinary skill in the art may make some modifications and improvements without departing from the concept of the present disclosure, and these all fall within the protection scope of the present disclosure. Therefore, the protection scope of the disclosure patent shall be subjected to the appended claims.

## Claims

1. A surgical navigation method, **characterized by** comprising:
performing optical tracking on an ultrasound probe and a puncture needle configured with optical markers to acquire three-dimensional tracking information of the ultrasound probe and the puncture needle relative to a first coordinate system;
acquiring ultrasound imaging data collected in real time by the ultrasound probe, wherein the ultrasound imaging data comprises an ultrasound image relative to a second coordinate system;
according to the ultrasound image relative to the second coordinate system and the three-dimensional tracking information of the ultrasound probe and the puncture needle relative to the first coordinate system, generating a position and a trajectory of the puncture needle in the ultrasound image in the second coordinate system; and
synchronously displaying the ultrasound image and the position and the trajectory of the puncture needle in the ultrasound image in the second coordinate system.

2. The surgical navigation method according to claim 1, **characterized in that**, before performing optical tracking on the ultrasound probe and the puncture needle configured with the optical markers, the method further comprising:
configuring the ultrasound probe and the puncture needle with the optical markers; and
acquiring three-dimensional calibration information of the ultrasound probe and the puncture needle configured with the optical markers; and
the performing optical tracking on the ultrasound probe and the puncture needle configured with the optical markers to acquire the three-dimensional tracking information of the ultrasound probe and the puncture needle relative to the first coordinate system comprising:
according to the three-dimensional calibration information of the ultrasound probe and the puncture needle configured with the optical markers, performing optical tracking on the ultrasound probe and the puncture needle configured with the optical markers to acquire the three-dimensional tracking information of the ultrasound probe and the puncture needle relative to the first coordinate system.

3. The surgical navigation method according to claim 1, **characterized in that**, the according to the ultrasound image relative to the second coordinate system and the three-dimensional tracking information of the ultrasound probe and the puncture needle relative to the first coordinate system, generating the position and the trajectory of the puncture needle in the ultrasound image in the second coordinate system comprising:
according to the ultrasound image relative to the second coordinate system and the three-dimensional tracking information of the ultrasound probe and the puncture needle relative to the first coordinate system, transforming the coordinates of the puncture needle into the ultrasound image in the second coordinate system; and
generating the position and the trajectory of the puncture needle in the ultrasound image in the second coordinate system.

4. The surgical navigation method according to claim 1, **characterized by** further comprising:
acquiring a mark of a target puncture area in the ultrasound image;
determining a shape of the target puncture area and a position of the target puncture area in the ultrasound image according to the mark; and
determining a distance between the puncture needle and the target puncture area in the ultrasound image according to the position of the target puncture area in the ultrasound image and the position and the trajectory of the puncture needle in the ultrasound image in the second coordinate system; and
the synchronously displaying the ultrasound image and the position and the trajectory of the puncture needle in the ultrasound image in the second coordinate system comprising:
synchronously displaying the ultrasound image, the shape of the target puncture area, the position of the target puncture area in the ultrasound image, the distance between the puncture needle and the target puncture area, and the position and the trajectory of the puncture needle in the ultrasound image.

5. The surgical navigation method according to claim 4, **characterized in that**, the position of the puncture needle in the ultrasound image in the second coordinate system comprising three-dimensional coordinates of the puncture needle in the ultrasound image in the second coordinate system, an offset distance of the puncture needle relative to the ultrasound image, and an intersection point between extended trajectory of the puncture needle and an ultrasound imaging plane.

6. The surgical navigation method according to claim 5, **characterized by** further comprising:
giving an alert if the offset distance of the puncture needle relative to the ultrasound image or the distance between the puncture needle and the target puncture area is smaller than a preset threshold.

7. A surgical navigation system, **characterized by** comprising:
a tracking module, configured to perform optical tracking on an ultrasound probe and a puncture needle configured with optical markers to acquire three-dimensional tracking information of the ultrasound probe and the puncture needle relative to a first coordinate system;
an ultrasound imaging data acquisition module, configured to acquire ultrasound imaging data collected in real time by the ultrasound probe, wherein the ultrasound imaging data comprises an ultrasound image relative to a second coordinate system;
a puncture needle trajectory generating module, configured to generate, according to the ultrasound image relative to the second coordinate system and the three-dimensional tracking information of the ultrasound probe and the puncture needle relative to the first coordinate system, a position and a trajectory of the puncture needle in the ultrasound image in the second coordinate system; and
a display module, configured to synchronously display the ultrasound image and the position and the trajectory of the puncture needle in the ultrasound image in the second coordinate system.

8. The surgical navigation system according to claim 7, **characterized by** further comprising:
an optical marker configuring module, configured to configure the ultrasound probe and the puncture needle with optical markers; and acquire three-dimensional calibration information of the ultrasound probe and the puncture needle configured with the optical markers.

9. The surgical navigation system according to claim 7, **characterized by** further comprising:
a target puncture area determining module, configured to acquire a mark of a target puncture area in the ultrasound image;
the target puncture area determining module is further configured to determine a shape of the target puncture area and a position of the target puncture area in the ultrasound image according to the mark; and
the target puncture area determining module is further configured to determine a distance between the puncture needle and the target puncture area in the ultrasound image according to the position of the target puncture area in the ultrasound image and the position and the trajectory of the puncture needle in the ultrasound image in the second coordinate system.

10. The surgical navigation system according to claim 9, **characterized in that**, the position of the puncture needle in the ultrasound image in the second coordinate system comprising an offset distance of the puncture needle relative to the ultrasound image and an intersection point between the extended trajectory of the puncture needle and an ultrasound imaging plane, and the surgical navigation system further comprising:
an alert module, configured to give an alert when the offset distance of the puncture needle relative to the ultrasound image or the distance between the puncture needle and the target puncture area is smaller than a preset threshold.
